# EUROPÄISCHE PATENTSCHRIFT

(11) **EP 2 077 150 B1**
(45) Veröffentlichungstag und Bekanntmachung des Hinweises auf die Patenterteilung: **16.03.2011**
(21) Anmeldenummer: 08021342.4
(22) Anmeldetag: 09.12.2008
(51) Int. Cl.: B01J 19/18, B01F 7/00, B29B 7/40, C07C 263/10

(54) **Verfahren und Mischaggregat zur Herstellung von Isocyanaten durch Phosgenierung primärer Amine**
Method and mixing unit for manufacturing isocyanates by phosgenesis of primary amines
Procédé et agrégat mixte destinés à la fabrication d'isocyanates par phosgénation d'amines primaires

(30) Priorität: 19.12.2007 DE 102007061688
(43) Veröffentlichungstag der Anmeldung: 08.07.2009
(73) Patentinhaber: Bayer MaterialScience AG, 51368 Leverkusen (DE)
(72) Erfinder: Rausch, Andreas, 41464 Neuss (DE); Steffens, Christian, Dr., 50737 Köln (DE); Wastian, Dietmar, 41542 Dormagen (DE); Keller-Killewald, Manfred, 41539 Dormagen (DE); Böhm, Matthias, 51373 Leverkusen (DE); Ritter, Joachim, Dr., 51373 Leverkusen (DE); Grünewald, Marcus, Dr., 44263 Dortmund (DE)

(56) Entgegenhaltungen:
- EP-A- 0 291 819
- EP-A- 0 830 894
- WO-A-98/33584

## Beschreibung

Die Erfindung betrifft ein Verfahren zur Herstellung von Isocyanaten durch Phosgenierung primärer Amine und ein für die Vermischung der Edukte Phosgen und primäres Amin geeignetes Mischaggregat vom Rotor-Stator-Typ. Das Mischaggregat vom Rotor-Stator-Typ ist ein Mischer-Reaktor und geeignet zur Vermischung, Einleitung und Durchführung einer Reaktion von mindestens zwei fließfähigen Stoffen, die erhebliche Viskositätsunterschiede aufweisen können, insbesondere zur Herstellung von Mono- oder Polyisocyanaten durch Umsetzung von Mono- oder Polyaminen mit in organischem Lösungsmittel gelöstem Phosgen.

Rotor-Stator-Mischer bestehen im Allgemeinen aus wenigstens einem mit Mischelementen versehenen Rotor und einem den Rotor umgebenden Stator, der mit strombrechenden Elementen ausgestattet ist. Derartige Rotor-Stator-Mischer sind allgemein bekannt. Der Rotor rotiert dabei mit hoher Drehzahl während der Stator ortsfest bleibt. Durch die Bewegung des Rotors wird die Flüssigkeit, die sich in dem Ringspalt zwischen dem Rotor und dem Stator befindet, turbulent stark verwischt. Bei nichtmischbaren Flüssigkeiten wird durch den hohen Energieeintrag eine der beiden Flüssigkeiten in der anderen feinteilig dispergiert. Dabei ist die entstehende Dispersion umso feinteiliger je höher die Drehzahl und damit die eingebrachte Energie ist. Aufgrund der hohen Drehzahl des Rotors wird während des Mischens viel Energie in die Flüssigkeit eingebracht und in Wärme umgewandelt. Dadurch kommt es im Verlauf des Mischvorgangs zu einer Temperaturerhöhung der flüssigen Mischung.

Es ist bekannt, schnell einsetzende Reaktionen, wie die Herstellung von Mono- oder Polyisocyanaten durch Umsetzung von Mono- oder Polyaminen mit Phosgen in einem Mischer-Reaktor vom Rotor-Stator-Typ (und ggf. zusätzlichen nachgeschalteten Reaktionsapparaten) durchzuführen, der aus einem im Wesentlichen rotationssymmetrischen Gehäuse besteht, wobei das Gehäuse eine im Wesentlichen rotationssymmetrische Mischkammer mit getrennten Einlässen für mindestens zwei Stoffe und einen Auslass aufweist, wobei der Einlass für den ersten Stoff in der Achse der Mischkammer vorgesehen ist und der Einlass für den mindestens zweiten Stoff in Form einer Vielzahl von rotationssymmetrisch zur Mischkammerachse angeordneten Öffnungen ausgebildet ist (EP 291 819 B1, EP 291 820 B1).

Bevorzugt kommen dabei nach dem Stand der Technik Mischaggregate zum Einsatz, in denen jedem Einlass, der in Form einer rotationssymmetrisch zur Mischkammerachse angeordnete Öffnungen ausgebildet ist, ein in axialer Richtung verschiebbarer Bolzen zugeordnet ist, mit dem die Öffnung im Falle eines Druckanstieges in der Zufuhrleitung oder periodisch durchstoßen und somit von eventuell vorhandenen Ablagerungen befreit werden kann (EP 830 894 B1).

Es ist ebenfalls bekannt, schnell einsetzende Reaktionen wie die Umsetzung von Mono- oder Polyaminen mit Phosgen in Mischaggregaten durchzuführen, die aus einem im Wesentlichen rotationssymmetrischen Gehäuse bestehen, wobei das Gehäuse eine im Wesentlichen rotationssymmetrische Mischkammer mit getrennten Einlässen für mindestens zwei Stoffe und einen Auslass aufweist, wobei der Einlass für den ersten Stoff in der Achse der Mischkammer vorgesehen ist und der Einlass für den mindestens zweiten Stoff radial oder seitlich zur Achse der Mischkammer erfolgt und die Mischkammer keine beweglichen Teile aufweist (EP 322 647 B1, WO 2002/002217 A1).

Es ist darüber hinaus bekannt, schnell einsetzende Reaktionen wie die Umsetzung von Mono- oder Polyaminen mit Phosgen in Mischaggregaten durchzuführen, die aus einem im Wesentlichen rotationssymmetrischen Gehäuse bestehen, wobei das Gehäuse eine im Wesentlichen rotationssymmetrische Mischkammer mit getrennten Einlässen für mindestens zwei Stoffe und einen Auslass aufweist, wobei mindestens beide Einlässe radial zur Achse der Mischkammer angeordnet sind (DE 10 034 621 A1, US-A 488 6368, DE 42 20 239 C2)

Die Qualität von in derartigen Apparaten hergestellten Mono- oder Polyisocyanaten hängt entscheidend von der Güte und Geschwindigkeit der Vermischung der mindestens zwei fließfähigen Stoffe ab. Dabei spielt insbesondere die Einhaltung eines gleichmäßigen Massenstromes durch den Mischer-Reaktor hindurch eine entscheidende Rolle, da dadurch eine Rückvermischung bereits miteinander reagierter Stoffe in die Stoffströme der unreagierten Einsatzstoffe verhindert werden kann.

Als generelles Kriterium für die Güte eines Mischapparates gilt die Mischzeit, die mit diesem erzielt werden kann. Die Mischzeit einer Mischeinrichtung, die zur Einleitung einer schnellen Reaktion wie der Herstellung von Mono- oder Polyisocyanaten durch Umsetzung von Mono- oder Polyaminen mit in organischem Lösungsmittel gelöstem Phosgen eingesetzt wird, beträgt üblicherweise 0,0001 s bis 5 s, bevorzugt 0,0005 bis 4 s, besonders bevorzugt 0,001 s bis 3 s (DE 10 2005 014846 A1). Als Mischzeit ist diejenige Zeit zu verstehen, die von dem Beginn des Mischvorgangs vergeht, bis 97,5 % der Fluidelemente des erhaltenen Gemisches einen Mischungsbruch erreicht haben, der bezogen auf den Wert des theoretischen Endwertes des Mischungsbruchs des erhaltenen Gemisches beim Erreichen des Zustandes perfekter Mischung weniger als 2,5 % von diesem Endwert des Mischungsbruches abweicht. Das Konzept des Mischungsbruches ist erläutert z.B. in J. Wamatz, U. Maas, R.W. Dibbla: Verbrennung, Springer Verlag, Berlin Heidelberg New York, 1997, 2. Auflage, S. 134.

Die Güte der Durchmischung und die Vollständigkeit der Verhinderung von Rückvermischung kann konkret an mehreren Kriterien erkannt werden:

Durch unzureichende Vermischung kommt es innerhalb der Einlassöffnungen für den mindestens zweiten Stoff im Laufe der Zeit zu Anbackungen bis hin zu Verstopfungen, so dass die Einleitung gleicher Massenströme durch alle Öffnungen gestört wird. Dies beeinträchtigt das Fließverhalten durch den Mischer-Reaktor hindurch, so dass es verstärkt zu Rückvermischung kommt.

Als weiteres Kriterium für die Güte der Durchmischung gelten Größe und Größenverteilung der Aminhydrochlorid- und Carbamoylchloridpartikel, die sich während der Reaktion bilden und deren Größe im Nanometer- bis Mikrometerbereich liegen sollte. Die Entstehung größerer Mengen dieser Feststoffe ist zu verhindern, da es dadurch zur Bildung großer und agglomerierter Aminhydrochloridpartikel kommen kann, deren Phosgenierung, wie in der Literatur beschrieben, sehr langsam ist (WO 2004/056756 A1).

Als weiteres Kriterium für die Güte der Durchmischung gilt auch die Farbe oder die Viskosität des erhaltenen Mono- oder Polyisocyanates, da bei vollständiger Unterdrückung aller Nebenreaktionen ein farbloses und niedrigviskoses Produkt erhalten werden kann.

Als weiteres Kriterium für die Güte der Durchmischung gilt der Gehalt an freien Isocyanatgruppen (NCO-Wert) im erhaltenen Produkt, da der Gehalt bei unzureichender Durchmischung klein bleibt, und bei vorhandener Rückvermischung weiter sinkt. Der Gehalt an freien Isocyanatgruppen kann als sogenannter NCO-Wert einfach bestimmt werden. Die Bestimmung des NCO-Wertes erfolgt durch Umsetzung des Isocyanates mit überschüssigem Dibutylamin zum korrespondierenden Harnstoff und Rücktitration des nicht verbrauchten Amins mit Salzsäure-Maßlösung. Ein hoher NCO-Wert wird für die technische Anwendung der Mono- und Polyisocyanate bevorzugt.

In den bekannten Mischaggregaten vom Rotor-Stator-Typ erfolgt die Mischung auf die Weise, dass der axial dosierte erste Stoff durch die Fliehkraft der ersten Rotorscheibe nach außen abströmt und dabei mit dem eingeführten zweiten Stoff beaufschlagt wird und durch die Rotationskomponente beide Stoffströme miteinander vermischt werden.

Bei der Herstellung von Mono- oder Polyisocyanaten durch Umsetzung von Mono- oder Polyaminen mit in organischem Lösungsmittel gelöstem Phosgen mittels eines Mischaggregats vom Rotor-Stator-Typ wird die Phosgenlösung bevorzugt axial zur Mischkammerachse dosiert und die Aminlösung durch die rotationssymmetrisch angeordneten Einlassöffnungen. Dies rührt daher, dass der Einlass der Aminlösung anfälliger für Verstopfungen ist und daher die Aminlösung bevorzugt durch die Einlassöffnungen dosiert wird, denen je ein Bolzen zugeordnet ist, mit dem die Ablagerungen entfernt werden können.

Nachteilig an den bekannten Mischer-Reaktoren vom Rotor-Stator-Typ ist, dass sich zwei Lösungen mit Viskositäten deren Verhältnis kleiner als 0,5 oder größer als 2 ist, nicht mehr ausreichend mischen lassen, wenn der Stoff mit der niedrigeren Viskosität axial zur Mischkammerachse dosiert wird, da seine durch die erste Rotorscheibe übertragene Fliehkraft nicht mehr ausreicht um den aus den rotationssymmetrisch angeordneten Öffnungen austretenden zweiten Stoff höherer Viskosität in Richtung des Auslasses der Mischkammer zu verdrängen. Dadurch kommt es zu Rückvermischung, die in der Mischkammer - das ist insbesondere an der Stirnplatte des Gehäuses und an den Gehäusewänden zwischen den Statoren - zu Anbackungen von Feststoffen und in den erhaltenen Mono- oder Polyisocyanaten zu einem niedrigen Gehalt an freien Isocyanatgruppen führt.

Nachteilig an den bekannten Mischer-Reaktoren vom Rotor-Stator-Typ ist darüber hinaus, dass die Konzentrationen der gelösten Stoffe nicht beliebig gewählt werden können, sondern die Konzentration der höherviskosen Lösung dadurch limitiert ist, dass ihre Viskosität nicht mehr als das doppelte der Viskosität der mindestens zweiten Lösung betragen darf. Dies ist insbesondere nachteilig bei der Herstellung von Mono- oder Polyisocyanaten, durch Umsetzung von Mono- oder Polyaminen mit Phosgen in organischen Lösungsmitteln, da die Viskosität der Mono- oder Polyaminlösung sich sehr stark mit der Konzentration des Mono- oder Polyamins in der Lösung ändert, während die Viskosität der Phosgenlösung mit verschiedenen Phosgenkonzentration nur geringfügig ansteigt. So liegt die Viskosität von Lösungen von Phosgen in Monochlorbenzol (MCB) im Konzentrationsbereich von 0 bis 80 Gew.-% bei 0°C zwischen 0,5 und 1,0 mPa s (Viskosität 0,765 mPas und Dichte 1,27 g/L bei 0°C und 50 bzw. 56 Gew.-%), während die Viskosität einer Lösung von Methylendiphenyldiamin (MDA) in Monochlorbenzol im Konzentrationsbereich von 15 bis 65 Gew.-% bei 25°C zwischen 1 und 200 mPa s liegt (Tabelle 1). Der Dichteunterschied der Lösungen ist hingegen nur gering und wirkt sich nicht erschwerend auf die Mischaufgabe aus.

**Tabelle 1: Dichte und Viskositäten verschiedener MDA in MCB-Lösungen bei 25°C, bestimmt mit einem Kugelfallviskosimeter nach Höppler der Fa. Haake nach DIN 53015**

| Konzentration MDA in MCB in % | Temperatur in °C | Dichte in g/ml | Viskosität in mPa·s |
|---|---|---|---|
| 15 | 25 | 1,10 | 0,99 |
| 30 | 25 | 1,10 | 1,89 |
| 45 | 25 | 1,10 | 4,29 |
| 50 | 25 | 1,10 | 5,40 |
| 65 | 25 | 1,14 | 20,98 |
| 95 | 25 | 1,20 | > 200 |

Die Aufgabe besteht somit darin, einen Mischer-Reaktor bereit zu stellen, der die genannten Nachteile umgeht, und der auch für zwei fließfähige Stoffe mit stark unterschiedlichen Viskositäten eine Durchmischung in einer Güte und Geschwindigkeit gewährleistet, die ein Verfahren zur Herstellung von Mono- oder Polyisocyanaten mit einem hohen Gehalt an freien Isocyanatgruppen erlaubt und somit den Einsatz von hochkonzentrierten Amin- und Phosgenlösungen ermöglicht.

Die Erfindung betrifft einen Mischer-Reaktor vom Rotor-Stator-Typ umfassend ein im Wesentlichen rotationssymmetrisches Gehäuse, das stirnseitig von einer Stirnplatte begrenzt wird, und welches eine Mischkammer mit getrennten Einlässen für mindestens zwei Stoffe und einen Auslass enthält, wobei der Einlass für den ersten Stoff bevorzugt in der Rotationsachse der Mischkammer vorgesehen ist und wobei der Einlass für den mindestens zweiten Stoff in Form einer Vielzahl von rotationssymmetrisch zur Rotationsachse angeordneten Öffnungen in der Stirnplatte ausgebildet ist, denen jeweils ein in axialer Richtung verschiebbarer Bolzen zugeordnet ist, dadurch gekennzeichnet, dass in der Stirnplatte Kanäle angeordnet sind, die von dem Einlass für den ersten Stoff in der Rotationsachse nach außen hin ausgerichtet sind.

Der erfindungsgemäße Mischer-Reaktor ist geeignet zur Vermischung und Durchführung bzw. Einleitung einer Reaktion von mindestens zwei fließfähigen Stoffen. Bevorzugt wird er eingesetzt für die Vermischung mindestens zweier fließfähiger Stoffe, Suspensionen oder Lösungen, bei denen das Verhältnis der Viskositäten des ersten Stoffes und des mindestens zweiten Stoffes bei Eintritt in den Mischer-Reaktor kleiner als 0,5 oder größer als 2 ist (Bestimmung der Viskosität mit einem Kugelfallviskosimeter nach Höppler der Fa. Haake nach DIN 53015). Geeignet ist der Mischer-Reaktor insbesondere für die Vermischung und Durchführung bzw. Einleitung einer Phosgenierungs-Reaktion, wenn als erster Stoff in einem Lösungsmittel gelöstes Phosgen und als zweiter Stoff eine Lösung eines primären Amins eingesetzt wird.

Die Kanäle führen bevorzugt von dem Einlass für den ersten Stoff in der Mischkammerachse (Rotationsachse) in der Stirnplatte radial nach außen. Bevorzugt enden die Kanäle in dem Abstand von der Rotationsachse, in dem auch die am weitesten außen liegenden Einlassöffnungen für den mindestens zweiten Stoff angeordnet sind. Die Kanäle sind als Vertiefungen in der Stirnplatte oder als aufgesetzte Führungen ausgeführt und können beliebige Formen haben, also beispielsweise dreieckigen, rechteckigen, halbrunden oder ovalen Querschnitt aufweisen. Die Kanäle sind mindestens am Anfang, also an der Stelle des Einlasses für den ersten Strom und mindestens an Ihrem Ende, also auf dem in radialer Richtung am weitesten außen liegenden Ort der Einlassöffnungen für den zweiten Strom, offen. Der dazwischen liegende Bereich der Kanäle kann offen oder geschlossen, d.h. in Richtung der Mischkammer hin durch Abdeckungen (beispielsweise durch Platten) verdeckt und nur in Strömungsrichtung parallel zur Ebene der Stirnplatte hin offen, sein. Bevorzugt sind die Kanäle in dem dazwischen liegenden Bereich geschlossen bzw. verdeckt, weil dann die Vermischung noch schneller und besser erfolgt. Bevorzugt sind die Kanäle auf 5 bis 95% ihrer Länge, besonders bevorzugt auf 20 bis 90% ihrer Länge, ganz besonders bevorzugt auf 40 bis 85% ihr Länge geschlossen bzw. verdeckt. In einer bevorzugten Ausführungsform überdecken die Abdeckungen die Kanäle bereits auf Höhe des Einlasses für den ersten Stoff, so dass der erste Stoff die Kanäle zwangsläufig durchströmen muss und dann die Kanäle durch die Kanalöffnungen für den ersten Strom wieder verlässt, in die Mischkammer gelangt und dort mit dem zweiten Stoff vermischt wird.

Bevorzugt weist der Mischer-Reaktor zwischen 2 und 48 Kanäle auf. Weiterhin bevorzugt weist der Mischer-Reaktor bevorzugt zwischen 2 und 48 Öffnungen (Einlassöffnungen) für den zweiten Strom auf. Bevorzugt sind die Öffnungen (Einlassöffnungen) für den zweiten Strom auf einer, zwei oder drei konzentrischen Kreisen um die Rotationsachse angeordnet. Die Einlassöffnungen können aber auch auf noch mehr konzentrischen Kreisen um die Rotationsachse angeordnet sein.

Der erfindungsgemäße Mischer-Reaktor ist insbesondere geeignet als Phosgenierreaktor zur Herstellung von Mono- oder Polyisocyanaten. Dabei wird als erster Stoff in einem organischen Lösungsmittel gelöstes Phosgen und als mindestens zweiter Stoff gegebenenfalls in einem Lösungsmittel gelöstes primäres Mono- oder Polyamin eingesetzt.

Die Erfindung betrifft auch ein Verfahren zur Herstellung von Isocyanaten durch Phosgenierung primärer Amine, bei dem die primären Amine und Phosgen in dem erfindungsgemäßen Mischer-Reaktor vermischt und umgesetzt werden. Bevorzugt werden dabei das in einem Lösungsmittel gelöste Phosgen als erster Stoff und eine Lösung eines primären Amins als zweiter Stoff eingesetzt. Bevorzugt ist das Verhältnis der Viskositäten des ersten und des zweiten Stoffes bei Eintritt in den Mischer-Reaktor kleiner als 0,5. Die Bestimmung der Viskosität erfolgt dabei bevorzugt mit einem Kugelfallviskosimeter nach Höppler der Fa. Haake nach DIN 53015.

Geeignete Einsatzstoffe und Reaktionsbedingungen sind in EP 291 819 B1, EP 322 647 B1 und EP 1616 857 A1 offenbart.

Der erfindungsgemäße Mischer-Reaktor eignet sich zur Phosgenierung beliebiger primärer Mono- und Polyamine, insbesondere zur Herstellung der in der Polyurethanchemie üblichen organischen Polyisocyanate wie den Di- und Polyisocyanaten der Diphenylmethanreihe (MDI, monomeres MDI und/oder polymeres MDI), Toluylendiisocyanat (TDI), Xyloldiisocyanat (XDI), Hexamethylendüsocyanat (HDI), Isophorondiisocyanat (IPDI), oder Naphtalindiisocyanat. Bevorzugte Ausgangsmaterialien für das erfindungsgemäße Verfahren sind die 3 bis 95 gew.-%igen, vorzugsweise 20 bis 75 gew.-%igen Lösungen von Phosgen in geeigneten Lösungsmitteln sowie die 5 bis 95 gew.-%igen, vorzugsweise 20 bis 70 gew.-%igen Lösungen von Mono- oder Polyaminen in geeigneten Lösungsmitteln.

Geeignet Lösungsmittel zur Herstellung der Phosgen- und Aminlösung sind beliebige, unter den Reaktionsbedingungen inerte Lösungsmittel wie z.B. Chlorbenzol, ortho-Dichlorbenzol, Dioxan, Toluol, Xylol, Methylenchlorid, Perchlorethylen, Trichlorfluormethan oder Butylacetat.

Vorzugsweise werden Chlorbenzol oder ortho-Dichlorbenzol eingesetzt. Die Lösungsmittel können rein oder als beliebige Gemische der beispielhaft genannten Lösungsmittel eingesetzt werden. Zweekmäßigerweise wird man für die Aminkomponente und das Phosgen das gleiche Losungsmittel bzw. Lösungsmittelgemisch einsetzen, obwohl dies nicht unbedingt erforderlich ist.

In dem Mischer-Reaktor kommen die Phosgen- und Aminlösungen bevorzugt in solchen Mengen zum Einsatz, dass im Mischraum ein Molverhältnis von Phosgen : primären Aminogruppen von 1,1 : 1 bis 30 : 1, besonders bevorzugt von 1,25 : 1 bis 3 : 1, vorliegt.

Die eingesetzten Phosgen- und Aminlösungen können vor dem Einbringen in den Mischer-Reaktor temperiert werden. Üblicherweise weist die Phosgenlösung eine bevorzugte Temperatur von -50°C bis +80°C, besonders bevorzugt von -20°C bis +70°C, auf. Die Aminlösung kann auf eine bevorzugte Temperatur von +25°C bis +160°C, besonders bevorzugt +40°C bis +140°C temperiert werden. Ganz besonders bevorzugt liegt die Temperatur der Aminlösung zwischen +50 und +120°C. Bevorzugt erfolgt die Temperierung und Dosierung der Eduktlösungen bei einer Druckstufe, die oberhalb des Dampfdruckes der jeweiligen Lösung liegt. Bevorzugt werden die Phosgen- und Aminlösungen bei Temperaturen von 0°C bis +70°C bzw. +80°C und +120°C eingesetzt. Dabei können absolute Drücke von 1 bis 70 bar, bevorzugt 3 - 45 bar zur Anwendung kommen.

Für die Vermischung der Phosgen- und der Aminlösung in dem Mischer-Reaktor kann dieser beheizt, isoliert oder gekühlt werden, wobei er bevorzugt lediglich isoliert wird. Die Isolierung kann durch die verschiedenen in der Technik bekannten Methoden erfolgen und kann das Mischaggregat mit einbeziehen.

Die Erfindung wird nachfolgend anhand der beigefügten Figuren näher erläutert:
Figur 1 zeigt einen erfindungsgemäßen Mischer-Reaktor vom Rotor-Stator-Typ enthaltend eine Stirnplatte mit Kanälen.
Figur 2 zeigt eine Stirnplatte, die Bestandteil des erfindungsgemäßen Mischer-Reaktors ist.

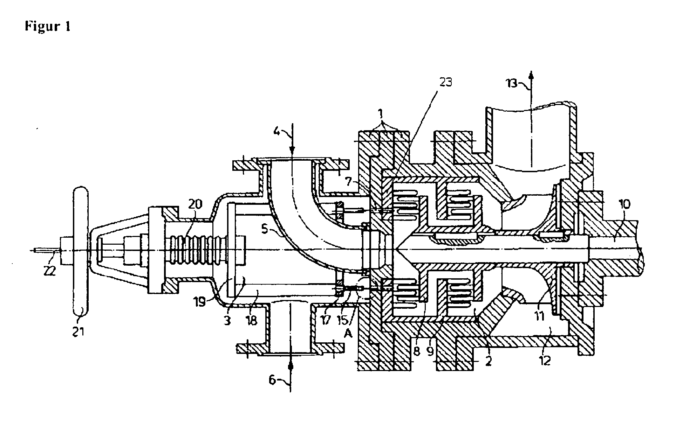

Der als axialer Schnitt dargestellte Mischer-Reaktor gemäß Figur 1 besteht aus einem Gehäuse 1, das eine Mischkammer 2 und eine Verteilkammer 3 aufweist. Der mindestens erste Stoffstrom 4 wird über ein in die Verteilkammerwand seitlich eintretendes gebogenes Rohr 5 axial in die Mischkammer 2 eingeleitet (und bildet den in der Rotationsachse 22 der Mischkammer 2 angeordneten Einlass für den ersten Stoff) und über Kanäle (nicht dargestellt in Figur 1) in der Stirnplatte 23 bis zu den Kanalöffnungen für den ersten Stoff (nicht dargestellt in Figur 1) geleitet. Der zweite Stoffstrom 6 wird in die Verteilkammer 3 eingeleitet und gelangt über eine Vielzahl von zur Achse des Mischer-Reaktors konzentrisch angeordneten parallelen Einlassöffnungen 7 in die Mischkammer 2. Die Mischkammer 2 enthält über eine Achse 10, die auf der Rotationsachse 22 angeordnet ist, angetriebene Rotorelemente 8 und mit dem Gehäuse verbundene Statorelemente 9. Ferner ist ein Laufrad 11 vorgesehen, das die Mischung über den Ringkanal 12 in das Auslassrohr 13 fördert. Jeder Einlassöffnung 7 sind Bolzen 15 zugeordnet, die auf einem Tragring 17 befestigt sind. Der Tragring 17 ist über Distanzstücke 18 mit einer Platte 19 verbunden, die über eine Achse mittels des Handrades 21 in axialer Richtung verschiebbar ist. Die Durchführung dieser Achse durch die Verteilkammerwand ist mittels Faltenbalg 20 gasdicht gekapselt.

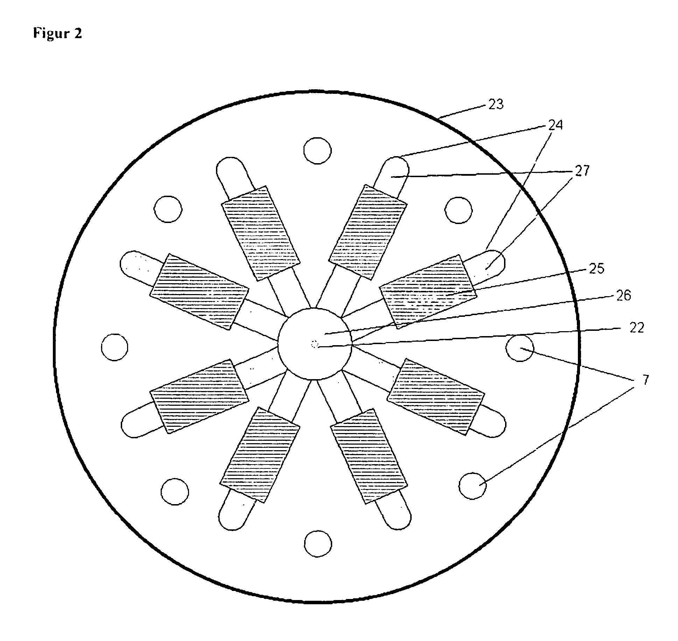

Figur 2 zeigt eine Stirnplatte 23, die Bestandteil des in Figur 1 gezeigten erfindungsgemäßen Mischer-Reaktors ist. Die Stirnplatte 23 weist einen Einlass 26 für den ersten Stoffstrom auf, sowie Kanäle 24, die diesen Stoff radial bis auf den Abstand der Einlassöffnungen 7 von der Rotationsachse 22 für den mindestens zweiten Strom weiterleiten. Die Kanäle 24 können als Vertiefungen in oder als Aufsätze auf der Stirnplatte 23 angebracht werden. Sie können vollständig geöffnet sein, sind aber bevorzugt im Bereich von 5 bis 95% ihrer Länge mit Abdeckungen 25 ganz oder teilweise zur Mischkammer hin verdeckt. Der erste Stoffstrom wird dann durch den Einlass 26 für den ersten Stoffstrom geleitet, strömt durch die mit den Abdeckungen 25 abgedeckten Kanäle 24 und verlässt die Kanäle anschließend durch die Kanalöffnungen 27 für den ersten Strom. In einer bevorzugten Ausführungsform (nicht gezeigt in Figur 2) überdecken die Abdeckungen 25 die Kanäle 24 bereits auf Höhe des Einlasses 26 für den ersten Stoff, so dass der erste Stoffstrom die Kanäle zwangsläufig durchströmen muss und dann die Kanäle durch die Kanalöffnungen 27 für den ersten Strom wieder verlässt und in die Mischkammer eindringt.

## Patentansprüche

1. Mischer-Reaktor vom Rotor-Stator-Typ umfassend ein im Wesentlichen rotationssymmetrisches Gehäuse (1), das stirnseitig von einer Stirnplatte (23) begrenzt wird, und welches eine Mischkammer (2) mit getrennten Einlässen für mindestens zwei Stoffe (4,6) und einen Auslass (13) enthält, wobei der Einlass (26) für den ersten Stoff (4) in der Rotationsachse (22) der Mischkammer (2) vorgesehen ist und wobei der Einlass für den mindestens zweiten Stoff (6) in Form einer Vielzahl von rotationssymmetrisch zur Rotationsachse angeordneten Öffnungen (7) in der Stirnplatte (23) ausgebildet ist, denen jeweils ein in axialer Richtung verschiebbarer Bolzen (15) zugeordnet ist, **dadurch gekennzeichnet, dass** in der Stirnplatte (23) Kanäle (24) angeordnet sind, die von dem Einlass (26) für den ersten Stoff in der Rotationsachse (22) nach außen hin ausgerichtet sind.

2. Mischer-Reaktor nach Anspruch 1, **dadurch gekennzeichnet, dass** die Kanäle in der Stirnplatte von dem Einlass für den ersten Stoff in der Rotationsachse in radialer Richtung bis zu dem Ort, an dem die am weitesten außen liegenden Öffnungen in der Stirnplatte für den mindestens zweiten Stoff angeordnet sind, ausgedehnt sind.

3. Mischer-Reaktor nach Anspruch 1 oder 2, **dadurch gekennzeichnet, dass** die Kanäle auf 5 bis 95 % ihrer Länge in Richtung der Mischkammer geschlossen sind.

4. Verwendung des Mischer-Reaktors nach einem der Ansprüche 1 bis 3 für die Vermischung mindestens zweier fließfähiger Stoffe, Suspensionen oder Lösungen.

5. Verwendung nach Anspruch 4, **dadurch gekennzeichnet, dass** das Verhältnis der Viskositäten des ersten Stoffes und des mindestens zweiten Stoffes bei Eintritt in den Mischer-Reaktor kleiner als 0,5 oder größer als 2 ist.

6. Verwendung nach Anspruch 4 oder 5, **dadurch gekennzeichnet, dass** als erster Stoff in einem Lösungsmittel gelöstes Phosgen und als zweiter Stoff eine Lösung eines primären Amins eingesetzt wird.

7. Verfahren zur Herstellung von Isocyanaten durch Phosgenierung primärer Amine, **dadurch gekennzeichnet, dass** die primären Amine und Phosgen in dem Mischer-Reaktor nach einem der Ansprüche 1 bis 3 vermischt und umgesetzt werden.

8. Verfahren nach Anspruch 7, **dadurch gekennzeichnet, dass** in einem Lösungsmittel gelöstes Phosgen als erster Stoff und eine Lösung eines primären Amins als zweiter Stoff eingesetzt wird und das Verhältnis der Viskositäten des ersten und des zweiten Stoffes bei Eintritt in den Mischer-Reaktor kleiner als 0,5 ist.

## Claims

1. Mixer-reactor of the rotor-stator type comprising a substantially rotationally symmetrical housing (1) which is bounded at the end by an end plate (23) and which comprises a mixing chamber (2) with separate inlets for at least two substances (4, 6) and an outlet (13), the inlet (26) for the first substance (4) being provided in the axis of rotation (22) of the mixing chamber (2), and the inlet for the at least one second substance (6) being configured in the form of a multitude of orifices (7) in the end plate (23) which are arranged in rotational symmetry with respect to the axis of rotation, to each of which is assigned a bolt (15) which is moveable in axial direction, **characterized in that** channels (24) arranged in the end plate (23) are aligned in the outward direction from the inlet (26) for the first substance in the axis of rotation (22).

2. Mixer-reactor according to Claim 1, **characterized in that** the channels in the end plate are extended from the inlet for the first substance in the axis of rotation in radial direction up to the point at which the orifices furthest to the outside in the end plate for the at least one second substance are arranged.

3. Mixer-reactor according to Claim 1 or 2, **characterized in that** the channels are closed for 5 to 95% of their length in the direction of the mixing chamber.

4. Use of the mixer-reactor according to any of Claims 1 to 3 for the mixing of at least two free-flowing substances, suspensions or solutions.

5. Use according to Claim 4, **characterized in that** the ratio of the viscosities of the first substance and of the at least one second substance on entry into the mixer-reactor is less than 0.5 or greater than 2.

6. Use according to Claim 4 or 5, **characterized in that** the first substance used is phosgene dissolved in a solvent, and the second substance used a solution of a primary amine.

7. Process for preparing isocyanates by phosgenating primary amines, **characterized in that** the primary amines and phosgene are mixed and converted in the mixer-reactor according to any of Claims 1 to 3.

8. Process according to Claim 7, **characterized in that** phosgene dissolved in a solvent is used as the first substance, and a solution of a primary amine as the second substance, and the ratio of the viscosities of the first and second substances on entry into the mixer-reactor is less than 0.5.

## Revendications

1. Mélangeur-réacteur de type rotor-stator comprenant un boîtier (1) essentiellement symétrique par rotation, qui est limité du côté frontal par une plaque frontale (23) et qui contient une chambre de mélange (2) munie d'entrées séparées pour au moins deux substances (4, 6) et d'une sortie (13), l'entrée (26) pour la première substance (4) étant prévue dans l'axe de rotation (22) de la chambre de mélange (2) et l'entrée pour la ou les secondes substances (6) étant conçue sous la forme d'une pluralité d'ouvertures (7) agencées selon une symétrie de rotation par rapport à l'axe de rotation dans la plaque frontale (23), auxquelles un boulon (15) mobile en direction axiale est attribué à chaque fois, **caractérisé en ce que** des canaux (24) sont placés dans la plaque frontale (23), qui sont orientés depuis l'entrée (26) pour la première substance dans l'axe de rotation (22) vers l'extérieur.

2. Mélangeur-réacteur selon la revendication 1, **caractérisé en ce que** les canaux dans la plaque frontale s'étendent depuis l'entrée pour la première substance dans l'axe de rotation en direction radiale jusqu'à l'emplacement où les ouvertures situées le plus à l'extérieur dans la plaque frontale pour la ou les secondes substances sont placées.

3. Mélangeur-réacteur selon la revendication 1 ou 2, **caractérisé en ce que** les canaux sont fermés sur 5 à 95 % de leur longueur dans la direction de la chambre de mélange.

4. Utilisation du mélangeur-réacteur selon l'une quelconque des revendications 1 à 3 pour le mélange d'au moins deux substances volatiles, suspensions ou solutions.

5. Utilisation selon la revendication 4, **caractérisée en ce que** le rapport entre les viscosités de la première substance et de la ou des secondes substances à l'entrée dans le mélangeur-réacteur est inférieur à 0,5 ou supérieur à 2.

6. Utilisation selon la revendication 4 ou 5, **caractérisée en ce que** du phosgène dissous dans un solvant est utilisé en tant que première substance et une solution d'une amine primaire en tant que seconde substance.

7. Procédé de fabrication d'isocyanates par phosgénation d'amines primaires, **caractérisé en ce que** les amines primaires et le phosgène sont mélangés et mis en réaction dans le mélangeur-réacteur selon l'une quelconque des revendications 1 à 3.

8. Procédé selon la revendication 7, **caractérisé en ce que** du phosgène dissous dans un solvant est utilisé en tant que première substance et une solution d'une amine primaire en tant que seconde substance et le rapport entre les viscosités de la première et de la seconde substance à l'entrée dans le mélangeur-réacteur est inférieur à 0,5.
